(19) Europäisches Patentamt
European Patent Office
Office européen des brevets

(11) EP 1 630 824 A1

(12) DEMANDE DE BREVET EUROPEEN

(43) Date de publication:
**01.03.2006 Bulletin 2006/09**

(51) Int Cl.:
***H01B 3/22*** *(2006.01)*

(21) Numéro de dépôt: **05077612.9**

(22) Date de dépôt: **19.09.1995**

(84) Etats contractants désignés:
**AT BE CH DE DK ES FR GB GR IE IT LI LU NL PT SE**

(30) Priorité: **30.09.1994 FR 9411718**

(62) Numéro(s) de document de la (des) demande(s) initiale(s) en application de l'article 76 CBE:
**95402099.6 / 0 704 861**

(71) Demandeur: **Arkema**
**92800 Puteaux (FR)**

(72) Inventeurs:
- **Berger, Noelle**
**69130 Ecully (FR)**
- **Commandeur, Raymond**
**38220 Vizille (FR)**

Remarques:
Cette demande a été déposée le 17 - 11 - 2005 comme demande divisionnaire de la demande mentionnée sous le code INID 62.

(54) **Composition dielectrique à base de polyarylalcanes ayant des proprietes dielectriques ameliorées**

(57) L'invention concerne l'utilisation pour les transformateurs de distribution dits "haute température" d'une composition diélectrique, comprenant au moins un oligomère de polyarylalcane, qui consiste en un isomère ou un mélange d'isomère de formule :

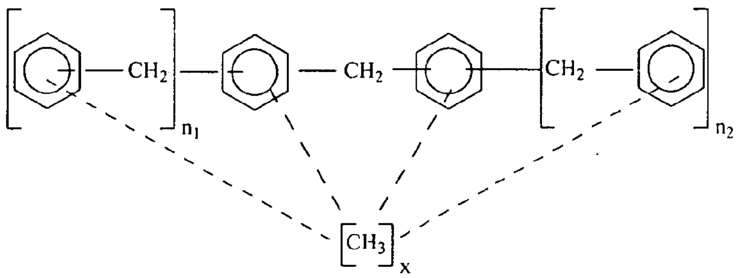

(I)

dans laquelle $n_1$ et $n_2$ = 0, 1 ou 2 sachant que $n_1 + n_2$ est égal à 1 ou 2, x = 0, 1, 2, 3, 4, 5 ou 6 et ayant notamment une tension de claquage augmentant avec la température.

EP 1 630 824 A1

## Description

La présente invention concerne une composition diélectrique à base de polyarylalcanes ayant des propriétés diélectriques améliorées et son utilisation dans les appareils électriques fonctionnant à haute température, notamment les transformateurs de distribution.

Une tendance actuelle est d'utiliser des transformateurs de distribution dont on s'efforce d'augmenter la puissance tout en conservant les mêmes dimensions.

Cette augmentation de puissance volumique entraîne une augmentation des températures de fonctionnement qui peuvent passer de 60°C - 80°C pour des transformateurs classiques, à 150°C voir 200°C pour les transformateurs de distribution dits "haute température".

Pour ce nouveau type d'appareil, on recherche des liquides qui possèdent de bonnes propriétés diélectriques même à haute température, une bonne stabilité thermique, une faible tension de vapeur et une viscosité suffisamment faible pour éliminer les calories.

Les huiles isolantes couramment utilisées telles que les huiles minérales, les huiles silicones, les esters de pentaerythritol possèdent un comportement à haute température relativement médiocre. Notamment il a été observé pour les huiles silicones un abaissement de la tension de claquage lorsque la température s'élevait (Etude effectuée par General Electric Co. pour le U.S. Department of Energy - rapport HCP/T-2115 publié en février 1979 - figure 3.3. page 35).

Ce comportement semble général car il a été également observé sur des liquides de synthèse tels que l'hexane (IEEE Trans. Electr. Insul. Vol El - 13 No4, August 1978 p263) et sur des huiles minérales ("Insulating Materials for Design and Pratice" de Franck M. Clark page 151- Ed. John Wiley and Sons, Inc).

Ceci présente notamment l'inconvénient de ne pouvoir garder les mêmes distances entre les conducteurs si l'on souhaite augmenter la puissance des appareils et, par conséquent, conduit à augmenter leur taille.

Par ailleurs, les huiles minérales et les huiles silicones présentent des stabilités thermiques limitées.

En outre, les huiles silicones sont de mauvais agents de transfert de chaleur.

On a maintenant trouvé une composition qui possède de meilleurs propriétés diélectriques, notamment, une tension de claquage qui augmente avec la température, une bonne stabilité thermique, une faible tension de vapeur et qui est également un bon agent de transfert de chaleur.

La composition selon l'invention est caractérisée en ce qu'elle comprend au moins un oligomère de polyarylalcane, qui consiste en un isomère ou un mélange d'isomères de formule :

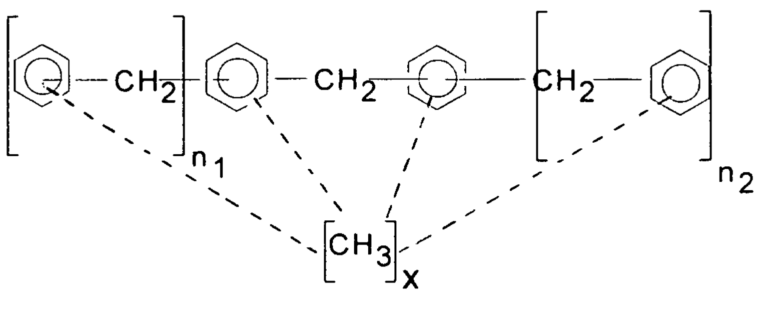

(I)

dans laquelle n1 et n2 = 0, 1 ou 2 sachant que n1 + n2 est égal à 1 ou 2
x = 0,1,2,3,4, 5 ou 6 et que ladite composition a

a) une tension de vapeur au plus égale à 1 torr à 150°C et, de préférence au plus égale à 0,25 torr,
b) un coefficient de qualité thermique défini comme le produit de la chaleur spécifique à 100°C (J/l/°C) par la conductivité thermique à 100°C (W/m/°C) divisé par la viscosité à 100°C ($mm^2/s$) et multiplié par 100 au moins égal à 3 et, de préférence au moins égal à 6,
c) un taux de formation de légers à 260°C pendant 500 heures au plus égal à 3g/kg et, de préférence, au plus égal à 1 g/kg,
d) une tension de claquage à 20°C au moins égale à 60kV, et de préférence au moins égale à 80 kV, et à 80°C, au moins égal à 110 kV et, de préférence au moins égale à 130 kV mesurée en courant alternatif de $50H_z$ dans une cellule équipée d'une électrode constituée par un barreau de diamètre égal à 0,6 mm et d'une électrode constituée par un disque Rogowski à bord arrondi de diamètre égal à 40 mm, lesdites électrodes étant séparées par une distances de 40 mm.

A titre d'illustration d'oligomère de formule (1) entrant dans la composition selon l'invention on peut citer :

♦ l'oligomère de formule (I) dans laquelle n1 = 1, n2 = 0 et x = 1

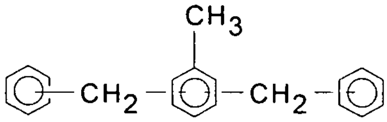

(Ia)

♦ l'oligomère de formule (I) dans laquelle n1 = 0, n2 = 1 et x = 1

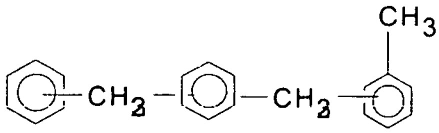

(Ib)

♦ l'oligomère de formule (I) dans laquelle $n_1$ et $n_2$ = 0, 1 ou 2 sachant que $n_1 + n_2 = 2$ et x = 1.

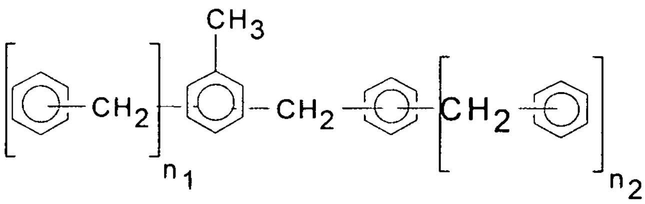

(Ic)

Selon la présente invention on préfère utiliser une composition comprenant une quantité pondérale d'oligomères (Ia) et (Ib) au moins égale à 40% et, de préférence une quantité pondérale allant de 70% à plus de 99% et une quantité pondérale d'oligomères (Ic) au plus égale à 3 % et, de préférence une quantité pondérale allant de 0,5 % à 2 %.

La composition de la présente invention peut éventuellement contenir de très faibles quantités d'oligomères de polyarylalcanes de formule (I) dans laquelle n1 = n2 = 0 et x = 1 (benzyltoluènes) voir même des oligomères de polyarylalcanes de formule (I) dans laquelle n1 = n2 = 0 et x = 0 (benzylbenzène).

Il est souhaitable que la quantité pondérale en benzyltoluènes et benzylbenzènes dans la composition soit aussi faible que possible, voir quasiment nulle car leur présence est de nature à augmenter notamment la tension de vapeur de ladite composition, ce qui est rédhibitoire pour l'utilisation envisagée.

Ces composés ont également l'inconvénient d'abaisser considérablement les points d'éclair et de feu des compositions les contenant. Ceci est rédhibitoire lorsque ces compositions doivent être utilisées dans des transformateurs de distribution haute température.

On ne sortirait pas du cadre de l'invention si l'oligomère de polyarylalcane de formule (I) était accompagné d'au moins un oligomère de polyarylalcane, consistant en un isomère ou mélange d'isomères de formule

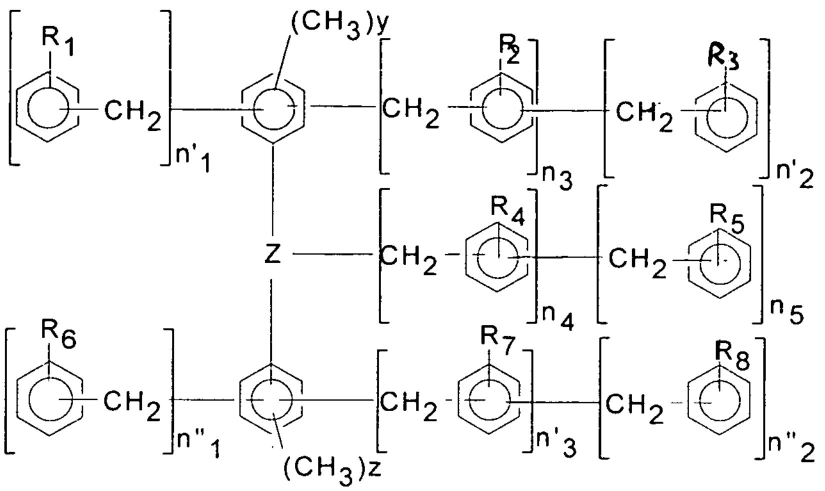

(II)

dans laquelle Z est un groupe de liaison trivalent tel que :

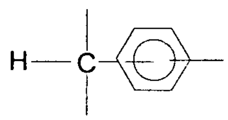

,

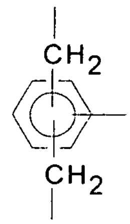

,

$R_1$, $R_2$, $R_3$, $R_4$, $R_5$, $R_6$, $R_7$, $R_8$ sont identiques au différents et représentent H ou $CH_3$,
n'1, n"1 et n4 = 0,1 ou 2 ;
n'2, n"2, n3, $n'_3$ et $n_5$ = 0 ou 1 ;
n'1 + n"1 + n'2 + n"2 + n'3 + n'3 + n4 + n5 $\leq$ 2,
y et z= 0, 1 ou 2

A titre d'illustration d'oligomères de formule (II), on peut citer les ditolylphénylméthanes :

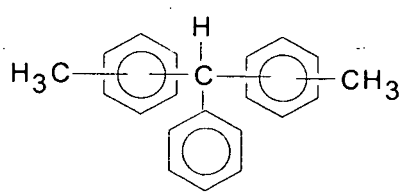

(IIa)

Selon la présente invention, la composition peut comprendre une quantité pondérale d'oligomères de formule (II) au plus égale à 10 %.

Selon une forme préférée de l'invention, la composition peut comprendre de :

. 60% à 70% en poids d'oligomères (Ia),

. 20% à 30% en poids d'oligomères (Ib),

. 0 % à 2 % en poids d'oligomères (Ic),

. 0% à 10% en poids de ditolylphenylméthanes (IIa)

Le total étant égal à 100%.

Les différents produits ou isomères de la composition selon l'invention, c'est-à-dire les oligomères de polyarylalcane de formule (I) et (II) peuvent être préparés par condensation du chlorure de benzyle ou de (méthyl)benzyle contenant une quantité variable de chlorure de benzylidène sur le toluène ou le xylène ou le mélange des deux en présence de catalyseur de Friedel et Crafts.

La condensation a lieu en pratique à une température comprise entre 50°C et 150°C.

La quantité de catalyseur est généralement comprise entre 50 ppm et 1% en poids de la masse réactionnelle.

A titre de catalyseur de Friedel et Crafts on peut utiliser des halogénures métalliques tels que le chlorure ferrique, le chlorure d'aluminium, le trichlorure d'antimoine, le tétrachlorure de titane.

Le mélange réactionnel obtenu est constitué essentiellement d'oligomères de polyarylalcane de formule (I) et (II) et de toluène non réagi.

Le mélange réactionnel peut subir un traitement de déchloration. A cet effet, on peut utiliser le procédé décrit dans EP 306398 utilisant un alcoolate de métal alcalin ou bien le procédé décrit dans EP 225849 utilisant le sodium métal, le contenu de ces demandes étant incorporé dans la présente invention.

Après le traitement de déchloration, on effectue une simple distillation, généralement sous pression réduite pour récupérer les oligomères de formule (I) et, éventuellement (II).

Les légers tels que les benzyltoluènes, le benzylbenzène sont avantageusement éliminés dans une première distillation, en même temps que le toluène ou le xylène non réagi et recyclés avant le traitement de déchloration.

On obtient en pied une fraction lourde pouvant contenir les restes de l'agent déchlorant, du NaCl, des résidus catalytiques et des oligomères de polyarylalcane plus lourds de formule (I) et / ou (II) comme par exemple les composés de formule (I) dans laquelle n1 + n2 = 2.

Les produits obtenus peuvent être purifiés selon une technique qui consiste à employer une terre de foulon ou d'alumine activée, soit seule, soit en mélange selon les techniques spécifiques connues dans le secteur des liquides diélectriques.

De même, il peut être avantageux d'ajouter des stabilisants du type époxyde ou d'autre nature, comme par exemple le tétraphénylétain ou des antioxydants.

Ces adjuvants sont généralement ajoutés en quantités variables entre 0,001% et 10%, de préférence entre 0,0 1 % et 0,3%.

Les compositions de l'invention sont avantageusement utilisées comme liquides diélectriques pour des isolations fonctionnant à haute température comme par exemple dans les transformateurs à puissance volumique améliorée.

Elles présentent en effet l'avantage d'avoir une tension de claquage qui augmentent avec la température contrairement aux liquides couramment utilisés tels que les huiles minérales, les huiles silicones ou les alkylbenzènes.

Ces compositions de l'invention possèdent par ailleurs une meilleur résistance aux décharges partielles. Ils présentent en outre une bien meilleure stabilité thermique.

Les compositions selon l'invention présentent également l'avantage de posséder des points d'éclair et des points de feu supérieurs à 200°C, qui est la température maximum d'utilisation.

Les exemples qui suivent illustrent l'invention.

**Exemple 1**

Dans un réacteur muni d'une agitation, d'un condenseur, d'un tube d'alimentation de chlore et d'une lampe PHILIPS TLADK de 30 Watts, on charge 40 moles de toluène. On introduit 10 moles de chlore gazeux en 4 heures à la température de 90°C. Le milieu réactionnel est dégazé, refroidi puis introduit progressivement dans un réacteur contenant 4 moles de toluène et 1g de chlorure ferrique à la température 90°C. En fin de réaction, la masse réactionnelle est dégazée puis soumise à une distillation avec une colonne de quatre plateaux sous une pression réduite de 100 mm de mercure de manière à éliminer le toluène non transformé.

On abaisse ensuite la pression vers 10 mm de mercure de manière à éliminer les isomères du benzyltoluène.

La température des vapeurs est de 140°C/150°C. La température en pied augmente à 240°C. Le mélange d'oligomères obtenu en pied est constitué d'oligomères de formule (I) dans laquelle n1 + n2 ≥ 1 avec x = 1 ainsi que des oligomères de formule (II).

Ce mélange est traité 6 heures à 300°C sous courant d'azote par 2% de méthylate de sodium puis il est soumis à une distillation sous pression réduite de 3 mm de mercure avec une colonne de 4 plateaux.

La fraction passant entre 200°C - 210°C a la composition pondérale suivante :

- 92,9% de (Ia) et (Ib)
- 4,1% d'oligomères de formule (II) dans laquelle

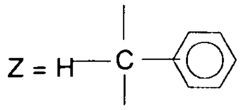

n'1 + n"1 + n'2+ n"2+ n3+ n'3+ n4+ n5=0 et y=z=3 (ditolylphénylméthanes)

- 1,9% de (Ic)
- moins de 1 % de benzyltoluènes (formule (I) dans laquelle n1 + n2 = 0 et x = 1)

La composition obtenue est celle qui sera utilisée dans tous les essais qui vont suivre.

♦ **Essai de détermination des tensions de claquage.**

Les mesures ont été effectuées en courant alternatif de 50 Hz en champ divergent (pointe 0,6 mm) dans une cellule telle que représentée sur la figure 1 avec pour électrodes :

⇒ un barreau de diamètre égal 0,6 mm (1)
⇒ un disque Rogowski bord arrondi, de diamètre égal à 40 mm (2) et deux volumes :

- un volume de 0,7 litre (3),
- un volume de 3 litres (4).

Pour chaque écartement entre les électrodes on a réalisé 5 à 6 mesures de la tension de claquage en appliquant une rampe de tension de 3000 V/s.

Dans une première série d'essais, nous avons effectué des mesures de tension de claquage à température ambiante (20°C), d'une part sur la composition de l'exemple 1, d'autre part sur différentes huiles minérales et sur du dodécylbenzène. Les résultats sont reportés sur le graphique 1. Sur ce graphique, nous avons représenté en ordonnée la tension de claquage en kV et en abscisse la distance entre les électrodes en mm.

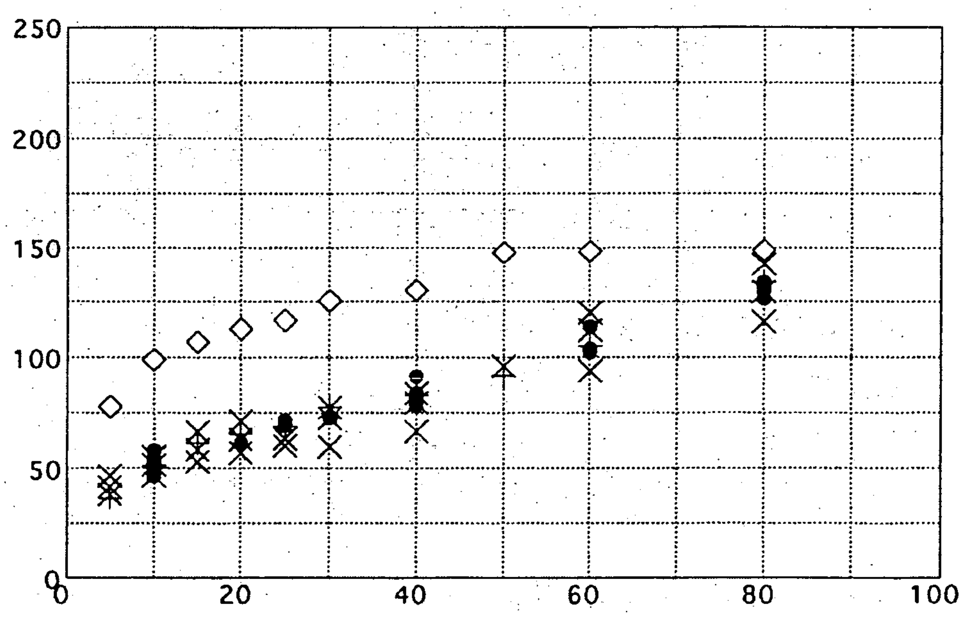


GRAPHIQUE 1

Dans ce graphique on représente également par :

● la composition selon l'exemple 1,

◇ le dodécylbenzène
x l'huile paraffinique dénommée Univolt 52,
+ l'huile minérale naphténique dénommée Nytro 10 G

On peut observer sur ce graphique que la composition selon l'exemple 1 présente systématiquement une tension de claquage égale à celle de l'huile minérale, qui augment lorsque l'écartement entre les électrodes augmente contrairement au dodécylbenzène (0) qui voit sa tension de claquage plafonner à partir d'un certain écartement.

Dans une seconde série d'essais nous avons effectué des mesures de tension de claquage à 20°C et à 80°C d'une part sur la composition de l'exemple 1, d'autre part sur du dodécylbenzène et sur l'huile minérale naphténique Nytro 10G

Les résultats sont reportés sur les graphiques 2 et 3.

Sur ces graphiques, nous avons représenté :

- en ordonnée, la tension de claquage en kV
- en abscisse, la distance entre les électrodes en mm

<u>**Sur le graphique 2**</u> - essais non conformes à l'invention, nous avons représenté par :

| | |
|---|---|
| Δ essais à 20°C | } pour le dodécylbenzène |
| ▲ essais à 80°C | } |
| □ essais à 20°C | } pour l'huile minérale naphténique Nytro 10G |
| ■ essais à 80°C | } |

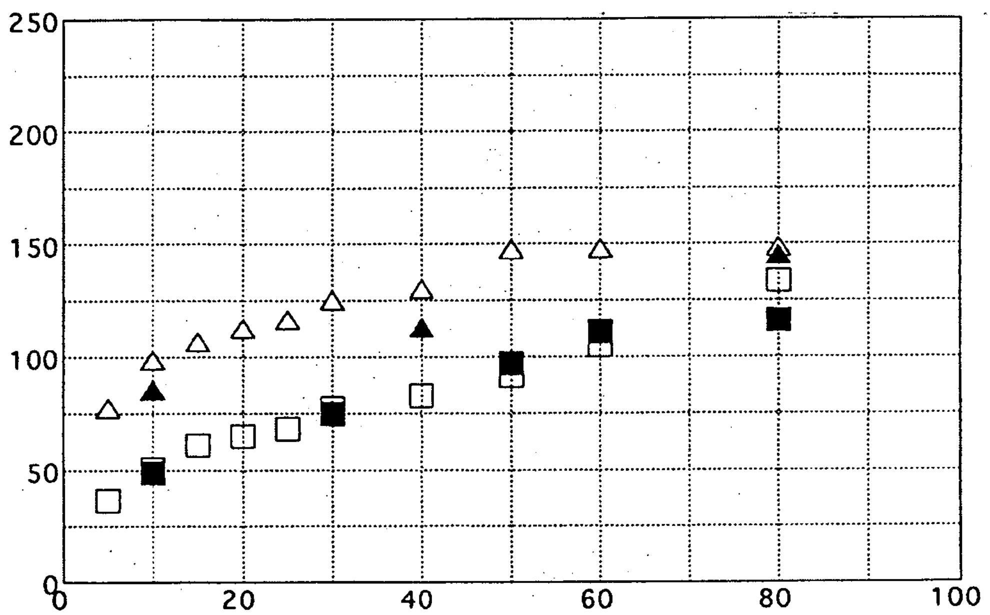


**GRAPHIQUE 2**

On peut observer sur ce graphique qu'il n'y a aucune différence de tension de claquage entre 20°C et 80°C, conformément à ce qui est connu.

Sur le graphique 3 (essais selon l'invention) nous avons représenté par:

□ essais à 20°C }
}sur la composition de l'exemple 1
■ essais à 80°C }

On observe une augmentation de la tension de claquage avec la température quelque soit l'écartement entre les électrodes. Par exemple, pour un écartement entre les électrodes égale à 40 mm on a une tension de claquage à 20°C de 80 kV et à 80°C de 130 kV.

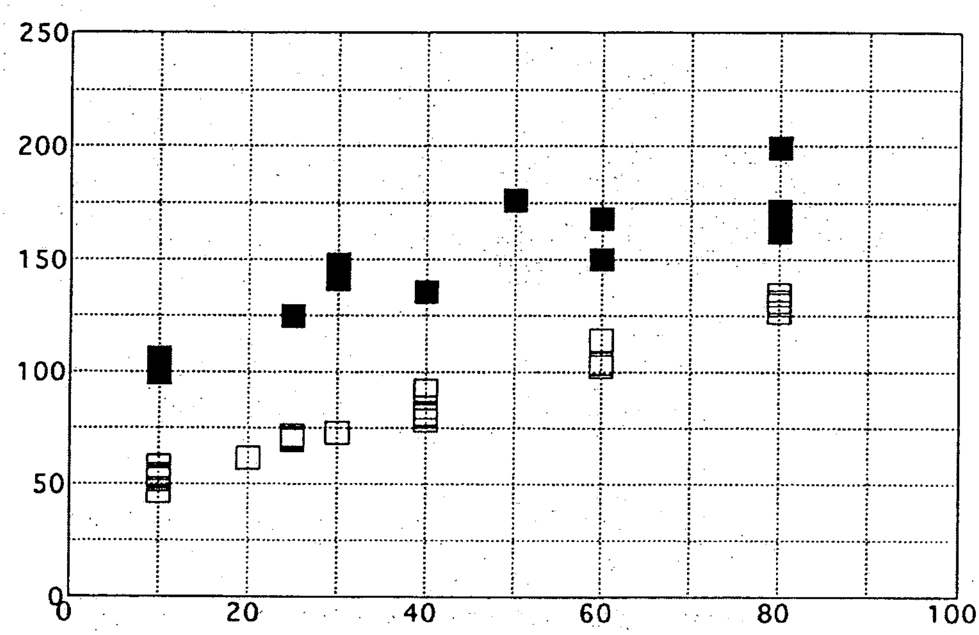


**GRAPHIQUE 3**

♦ **Essai de détermination de la tension de la vapeur**

Elle a été réalisée selon une méthode décrite dans :

Fluid Phase Equilibria, 42, pages 287 à 304 (1988).
Pour la composition de l'exemple 1, elle a été trouvée égale à 0,244 mm Hg à 150,9°C

♦ **Essai de détermination de légers à 260°C (tests de stabilité thermique)**

L'essai a été réalisé dans un appareillage tel que représenté sur la figure 2.
Les conditions de l'essai sont les suivantes :

- durée de l'essai : 500 heures
- température : 260°C
- on récupère les produits de décomposition condensables dans un condensateur à eau et les produits de décomposition non condensables sont piégés dans une cuve à eau.

On suit la décomposition thermique au cours du temps. Les résultats sont reportés sur les graphiques 4 et 5. Sur ces graphiques nous avons représenté en ordonnée le dégagement de produits légers condensables en g/kg. (graphique 4) et en litres/kg (graphique 5) et en abscisse le temps en heures.
Nous avons également représenté par :

● l'huile paraffinique UNIVOLT 52,
■ la composition de l'exemple 1,

- ♦ une huile silicone utilisée dans les transformateurs,
- ○ un esters de pentaerythritol dénomée MIDEL.

On constate que le taux de formation de produits légers condensables et non condensables est quasiment nul à 260°C après 500 heures.

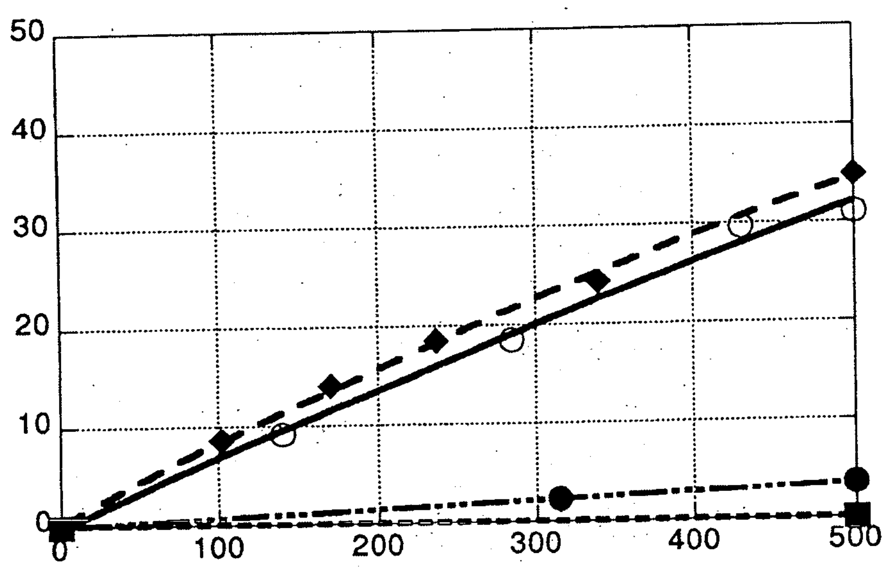


GRAPHIQUE 4

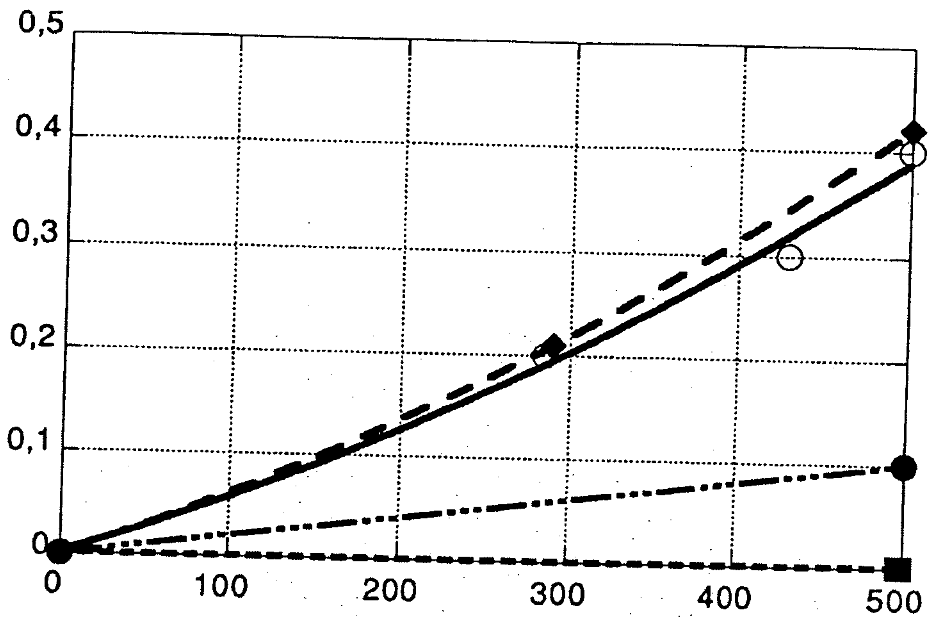


GRAPHIQUE 5

♦ **Essai de détermination du coefficient de qualité thermique**

On détermine la chaleur spécifique à 100°C par calorimétrie
On détermine la conductivité thermique à 100°C selon ASTM D 2717
On détermine la viscosité à 100°C selon ASTM D 445

Puis on applique la relation :

$$\frac{\text{chaleur spécifique X conductivité thermique}}{\text{Viscosité}} \quad \text{X } 100$$

Pour la composition de l'exemple 1, on a :

- chaleur spécifique à 100°C = 1,802 J/l/°C
- conductivité thermique à 100°C = 0,12146 W/m/°C
- viscosité thermique à 100°C = 3,1 $mm^2/s$

Dont le coefficient de qualité thermique est égal :

$$\text{Dont le coefficient de qualité thermique est égal : } \frac{1{,}802 \ \text{x} \ 0{,}12146}{3{,}1} \ \text{x } 100 = 7{,}06$$

**Revendications**

1. Utilisation pour les transformateurs de distribution dits "haute température" d'une composition diélectrique, comprenant au moins un oligomère de polyarylalcane, qui consiste en un isomère ou un mélange d'isomère de formule :

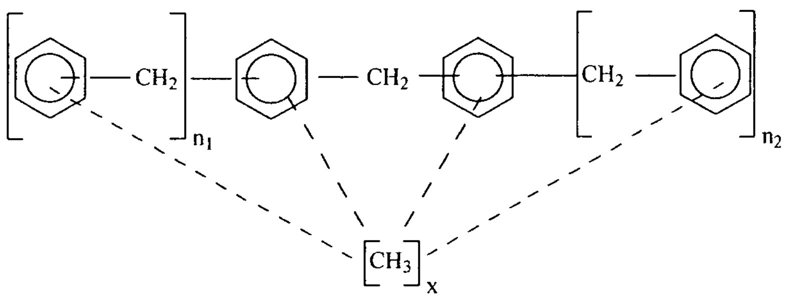

(I)

dans laquelle $n_1$ et $n_2$ = 0, 1 ou 2 sachant que $n_1 + n_2$ est égal à 1 ou 2, x = 0, 1, 2, 3, 4, 5 ou 6 et ayant :

a) une tension de vapeur au plus égale à 133,32 Pa à 150°C, mesurée selon la méthode décrite dans : 'Fluid Phase Equilibria, 42, pages 287 à 304 (1998),
b) un coefficient de qualité thermique défini comme le produit de la chaleur spécifique à 100°C (J/l/°C), mesurée par la méthode ASTM D 2766, par la conductivité thermique à 100°C (W/m/°C), mesurée par la méthode ASTM D 2717, divisé par la viscosité à 100°C ($mm^2/s$), mesurée par la méthode ASTM D 445, et multiplié par 100 au moins égal à 3,
c) un taux de formation de légers à 260°C pendant 500 heures au plus égal à 3 g/kg, mesuré dans un appareillage tel que représenté sur la figure 2, les conditions de l'essai étant les suivantes :

- durée de l'essai : 500 heures,
- température : 260 °C,
- on récupère les produits de décompositions condensables dans un condensateur à eau et les produits de décomposition non condensables sont piégés dans une cuve à eau,
- on suit la décomposition thermique au cours du temps,

d) une tension de claquage à 20°C au moins égale à 60 kV, et à 80°C, au moins égal à 110 kV mesurée en

courant alternatif de 50 $H_z$ dans une cellule équipée d'une électrode constituée par un barreau de diamètre égal à 0,6 mm et d'une électrode constituée par un disque Rogowski à bord arrondi de diamètre égal à 40 mm, lesdites électrodes étant séparées par une distance de 40 mm.

**2.** Utilisation d'une composition selon la revendication 1, **caractérisée en ce qu'**elle a :

a) une tension de vapeur au plus égale à 33,33 Pa à 150°C,
b) un coefficient de qualité thermique au moins égal à 6,
c) un taux de formation de légers à 260°C pendant 500 heures au plus égal à 1 g/kg, et
d) une tension de claquage à 20°C au moins égale à 80 kV, et à 80°C, au moins égal à 130 kV.

**3.** Utilisation d'une composition selon la revendication 1 ou 2, **caractérisée en ce qu'**elle comprend une quantité pondérale d'oligomères (Ia) -oligomères de formule (I) dans laquelle $n_1 = 1$, $n_2 = 0$ et $x = 1$ et d'oligomères (Ib) - oligomères de formule (I) dans laquelle $n_1 = 0$, $n_2 = 1$ et $x = 1$ au moins égale à 40 % et une quantité pondérale d'oligomères (Ic) - oligomères de formule (I) dans laquelle $n_1$ et $n_2 = 0$, 1 ou 2 sachant que $n_1 + n_2 = 2$ et $x = 1$ - au plus égale à 3 %.

**4.** Utilisation d'une composition selon la revendication 3, **caractérisée en ce qu'**elle comprend une quantité pondérale d'oligomères (Ia) et (Ib) allant de 70 % à plus de 99 % et une quantité pondérale d'oligomères (Ic) allant de 0,5 % à 2 %.

**5.** Utilisation d'une composition selon l'une quelconque des revendications 1 à 4, **caractérisée en ce qu'**elle comprend en outre au moins un oligomère de polyarylalcane qui consiste en un isomère ou mélange d'isomères de formule :

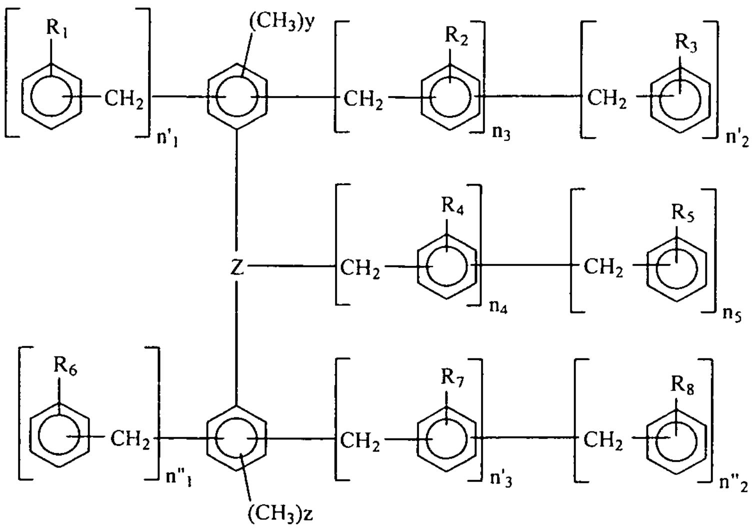

(II)

dans laquelle Z est un groupe de liaison trivalent tel que :

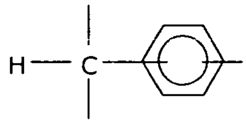

,

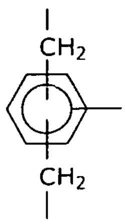

$R_1$, $R_2$, $R_3$, $R_4$, $R_5$, $R_6$, $R_7$, $R_8$ sont identiques ou différents et représentent H ou $CH_3$,
$n'_1$, $n''_1$ et $n_4$ =0,1 ou 2,
$n'_2$, $n''_2$, $n_3$, n'3 et $n_5$ = 0 ou 1,
$n'_1 + n''_1 + n'_2 + n''_2 + n_3 + n'_3 + n_4 + n_5 \leq 2$,

y et z=0, 1 ou2.

6. Utilisation d'une composition selon la revendication 5, **caractérisée en ce que** les oligomères de formule (II) sont les ditolylphénylméthanes de formule :

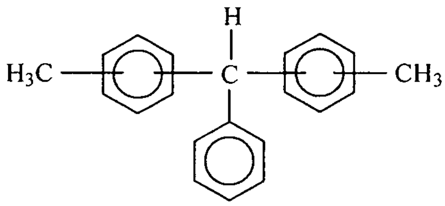

(IIa)

7. Utilisation d'une composition selon l'une quelconque des revendications 1 à 6, **caractérisée en ce qu'**elle comprend de :

- 60 % à 70 % en poids d'oligomères (Ia),
- 20 % à 30 % en poids d'oligomères (Ib),
- 0 % à 2 % en poids d'oligomères (1c),
- 0 % à 10 % en poids de ditolylphénylméthanes (IIa). Le total étant égal à 100 %.

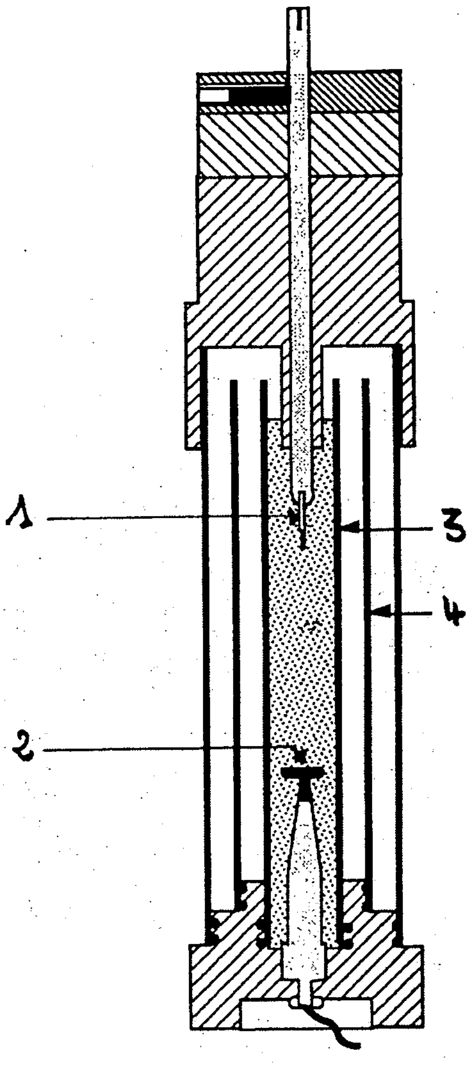


FIGURE 1

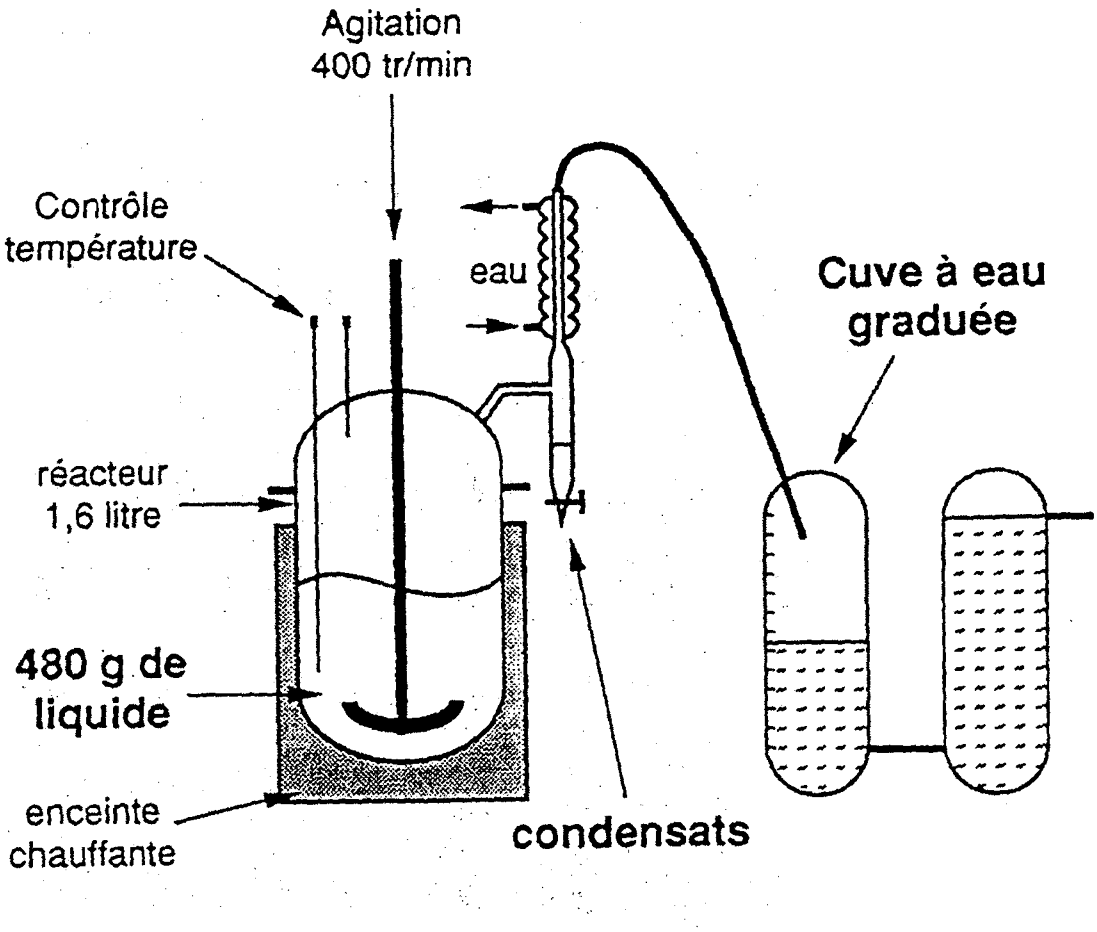
Agitation
400 tr/min
Contrôle
température
eau
Cuve à eau
graduée
réacteur
1,6 litre
480 g de
liquide
enceinte
chauffante
condensats

FIGURE 2

Office européen des brevets

# RAPPORT DE RECHERCHE EUROPEENNE

Numéro de la demande
EP 05 07 7612

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | EP 0 446 086 A (ELF ATOCHEM S.A) 11 septembre 1991 (1991-09-11) * le document en entier * ----- | 1-7 | H01B3/22 |
| A | EP 0 444 989 A (ELF ATOCHEM S.A) 4 septembre 1991 (1991-09-04) * le document en entier * ----- | 1-7 | |
| A | EP 0 544 571 A (ELF ATOCHEM S.A) 2 juin 1993 (1993-06-02) * le document en entier * ----- | 1-7 | |

DOMAINES TECHNIQUES RECHERCHES (IPC)

H01B

Le présent rapport a été établi pour toutes les revendications

| Lieu de la recherche | Date d'achèvement de la recherche | Examinateur |
|---|---|---|
| Munich | 4 janvier 2006 | Marquis, D |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.......................................................................................
& : membre de la même famille, document correspondant

2

EPO FORM 1503 03.82 (P04C02)

**ANNEXE AU RAPPORT DE RECHERCHE EUROPEENNE RELATIF A LA DEMANDE DE BREVET EUROPEEN NO.** EP 05 07 7612

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

04-01-2006

| Document brevet cité au rapport de recherche | Date de publication | Membre(s) de la famille de brevet(s) | Date de publication |
|---|---|---|---|
| EP 0446086 A | 11-09-1991 | BR 9100799 A | 05-11-1991 |
| | | CA 2036794 A1 | 28-08-1991 |
| | | CN 1054415 A | 11-09-1991 |
| | | DE 69100731 D1 | 20-01-1994 |
| | | DE 69100731 T2 | 19-05-1994 |
| | | DK 446086 T3 | 14-02-1994 |
| | | ES 2061193 T3 | 01-12-1994 |
| | | FI 910936 A | 28-08-1991 |
| | | FR 2658813 A1 | 30-08-1991 |
| | | IE 910654 A1 | 28-08-1991 |
| | | JP 4211620 A | 03-08-1992 |
| | | JP 7039355 B | 01-05-1995 |
| | | KR 9605273 B1 | 23-04-1996 |
| | | MX 172888 B | 17-01-1994 |
| | | NO 910754 A | 28-08-1991 |
| | | PT 96894 A | 31-10-1991 |
| | | ZA 9101400 A | 27-11-1991 |
| EP 0444989 A | 04-09-1991 | AT 111431 T | 15-09-1994 |
| | | BR 9100798 A | 05-11-1991 |
| | | CA 2037126 A1 | 28-08-1991 |
| | | CN 1054416 A | 11-09-1991 |
| | | DE 69103927 D1 | 20-10-1994 |
| | | DE 69103927 T2 | 04-05-1995 |
| | | DK 444989 T3 | 13-02-1995 |
| | | ES 2060311 T3 | 16-11-1994 |
| | | FI 910935 A | 28-08-1991 |
| | | FR 2658812 A1 | 30-08-1991 |
| | | IE 910614 A1 | 28-08-1991 |
| | | JP 4211619 A | 03-08-1992 |
| | | JP 7051520 B | 05-06-1995 |
| | | KR 9605274 B1 | 23-04-1996 |
| | | NO 910753 A | 28-08-1991 |
| | | PT 96893 A | 31-10-1991 |
| | | ZA 9101399 A | 27-11-1991 |
| EP 0544571 A | 02-06-1993 | AT 159042 T | 15-10-1997 |
| | | CA 2083752 A1 | 27-05-1993 |
| | | CN 1073967 A | 07-07-1993 |
| | | DE 69222616 D1 | 13-11-1997 |
| | | DE 69222616 T2 | 02-04-1998 |
| | | DK 544571 T3 | 04-05-1998 |
| | | ES 2106846 T3 | 16-11-1997 |
| | | FI 925351 A | 27-05-1993 |
| | | GR 3025827 T3 | 30-04-1998 |
| | | JP 6093278 A | 05-04-1994 |

EPO FORM P0460

Pour tout renseignement concernant cette annexe : voir Journal Officiel de l'Office européen des brevets, No.12/82

**ANNEXE AU RAPPORT DE RECHERCHE EUROPEENNE RELATIF A LA DEMANDE DE BREVET EUROPEEN NO.** EP 05 07 7612

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

04-01-2006

| Document brevet cité au rapport de recherche | Date de publication | Membre(s) de la famille de brevet(s) | Date de publication |
|---|---|---|---|
| EP 0544571 A | | JP 7078228 B | 23-08-1995 |
| | | NO 924400 A | 27-05-1993 |
| | | US 5446228 A | 29-08-1995 |

EPO FORM P0460

Pour tout renseignement concernant cette annexe : voir Journal Officiel de l'Office européen des brevets, No.12/82